# EUROPEAN PATENT APPLICATION

(11) **EP 4 751 659 A1**
(43) Date of publication of application: **03.06.2026**
(21) Application number: 25219582.1
(22) Date of filing: 28.11.2025
(51) Int. Cl.: A61B 8/12, A61B 8/00

(54) **INTERVENTIONAL ULTRASOUND IMAGING DEVICE**

(30) Priority: 29.11.2024 CN 202411748558
(71) Applicant: Wuhan United Imaging Healthcare Co., Ltd., WuHan, Hubei 430206 (CN)
(72) Inventor: YAO, Yuan, Wuhan, 430206 (CN); ZHU, Zhihao, Wuhan, 430206 (CN); KE, Changxing, Wuhan, 430206 (CN); SI, Kang, Wuhan, 430206 (CN)
(74) Representative: Murgitroyd & Company

(57) **Abstract**

An interventional ultrasound imaging device is disclosed, which includes an ultrasound probe (100) and a drive assembly (200). The ultrasound probe (100) includes a first connector (110), an inner sheath (120) and a housing (130) sequentially sleeved from inside to outside. The housing (130) is provided with a sliding member (140). The drive assembly (200) includes a second connector (210), a fixing member (220) and a guide member (230) sequentially sleeved from inside to outside. The guide member (230) is provided with a guide slot (234). The device has a separated state where the first connector (110) is separated from the second connector (210), and a locked state where the first connector (110) is electrically connected to the second connector (210) and the inner sheath (120) extends into the fixing member (220) partially and is connected to the fixing member (220). The sliding member (140) is configured to slide along the guide slot (234) to switch the device between the separated state and the locked state.

## Description

### TECHNICAL FIELD

The present disclosure relates to the field of medical instrument technologies, and in particular, to an interventional ultrasound imaging device.

### BACKGROUND

The interventional ultrasound imaging device includes an ultrasound probe and a drive assembly. The drive assembly is connected to the ultrasound probe through a butt joint structure. The ultrasound probe is driven by the drive assembly to perform rotary motion or linear motion, so as to adjust an ultrasound imaging range of the ultrasound probe. However, the conventional butt joint structure between the ultrasound probe and the drive assembly is complex in structure and inconvenient to operate.

### SUMMARY

An interventional ultrasound imaging device is provided in the present disclosure.

In some embodiments, an interventional ultrasound imaging device is provided. The interventional ultrasound imaging device includes: an ultrasound probe including a first connector, an inner sheath and a housing sequentially sleeved from inside to outside, the housing being provided with a sliding member; and a drive assembly including a second connector, a fixing member and a guide member sequentially sleeved from inside to outside, the guide member being provided with a guide slot. The device has a separated state where the first connector is separated from the second connector, and a locked state where the first connector is electrically connected to the second connector and the inner sheath extends into the fixing member partially and is connected to the fixing member. The sliding member is configured to slide along the guide slot to switch the device between the separated state and the locked state.

In one of the embodiments, a through hole is disposed on the fixing member, a movable member is movably disposed in the through hole, and a limiting groove is disposed on the inner sheath. When the device is in the locked state, the movable member is partially located in the through hole and partially located in the limiting groove to connect the fixing member and the inner sheath.

In one of the embodiments, the drive assembly includes a crimping member, the crimping member is disposed between the guide member and the fixing member, and the crimping member is configured to restrict the movable member from being disengaged from the through hole.

In one of the embodiments, the through hole includes a large end and a small end, the large end faces the crimping member, and the small end faces the second connector. A pressing portion is disposed on the crimping member, and the pressing portion is attached onto a side of the movable member away from the second connector, so that the movable member is partially disposed in the through hole and partially disposed in the limiting groove.

In one of the embodiments, the ultrasound probe is capable of approaching the drive assembly in a first direction to dock the ultrasound probe with the drive assembly, and the guide slot includes a first sliding portion extending parallel to the first direction. An abutment groove is disposed at an end of the crimping member close to the ultrasound probe, and a length of the first sliding portion in the first direction is greater than a length of an abutment groove in the first direction. The sliding member is capable of extending through the first sliding portion and the abutment groove simultaneously and abuts against a groove wall of the abutment groove, and the sliding member is capable of sliding along the first sliding portion to push the crimping member to move in the first direction, so that the pressing portion is separated from the movable member.

In one of the embodiments, the drive assembly further includes a reset member, a protrusion is disposed on the fixing member, the reset member is sleeved on the fixing member, an end of the reset member abuts against the pressing portion, and another end of the reset member abuts against the protrusion. The reset member is capable of pushing the crimping member to move in a second direction through an elastic force of the reset member, and the second direction is opposite to the first direction.

In one of the embodiments, the guide slot includes a second sliding portion communicating with the first sliding portion, and the second sliding portion extends in a circumferential direction of the guide member. An inclined surface groove communicating with an abutment groove is disposed on an outer peripheral surface of the crimping member, and a groove wall of the inclined surface groove extends from a groove wall of the abutment groove in a direction away from the ultrasound probe. The sliding member is capable of extending through the second sliding portion and the inclined surface groove simultaneously and sliding along the second sliding portion, so that the reset member pushes the pressing portion to move in the second direction.

In one of the embodiments, a projection of the inclined surface groove in an axial direction of the crimping member on a first projection plane forms a first arc, an arc length of the first arc is less than an arc length of the second sliding portion, and the first projection plane is a projection plane of the crimping member that is perpendicular to the axial direction of the crimping member.

In one of the embodiments. The ultrasound probe includes an outer sheath and a damping member. The outer sheath is connected to the housing. The damping member is sleeved on the inner sheath. And the outer sheath is connected to the inner sheath through the damping member.

In one of the embodiments, a recessed portion communicating with the inclined surface groove is disposed on an outer peripheral surface of the crimping member. The guide slot includes a third sliding portion communicating with the second sliding portion, and the third sliding portion extends from an end of the second sliding portion away from the first sliding portion in a direction close to the ultrasound probe. The sliding member extends through a third sliding portion and a recessed portion, and slides in a direction away from the second sliding portion to separate the damping member from the outer sheath.

In one of the embodiments, the sliding member extends through the third sliding portion and the recessed portion, and slides in a direction close to the second sliding portion to connect the damping member to the outer sheath.

In one of the embodiments, a length of the third sliding portion in the first direction is greater than or equal to a predetermined movement distance of the outer sheath.

In one of the embodiments, the drive assembly further includes a retaining ring, the retaining ring is sleeved on the fixing member and disposed on a side of the movable member close to the ultrasound probe, and the retaining ring abuts against the pressing portion when the pressing portion is attached onto the movable member.

In one of the embodiments, the ultrasound probe further includes a first rotating shaft extending through the inner sheath, the first connector is mounted on the first rotating shaft, and a first transmission member is mounted on the first rotating shaft. The drive assembly further includes a second rotating shaft extending through the fixing member, the second connector is mounted on the second rotating shaft, and a second transmission member is mounted on the second rotating shaft. When the device is in the locked state, the second transmission member is configured to contact the first transmission member, so that the drive assembly drives the ultrasound probe to rotate.

In one of the embodiments, the first transmission member has a first abutment plane, the second transmission member includes a second abutment plane, and the first abutment plane is configured to be attached onto the second abutment plane.

In one of the embodiments, the ultrasound probe further includes a bearing, and the first rotating shaft is rotatably connected to the inner sheath through the bearing.

In one of the embodiments, an inwardly recessed mounting cavity is disposed on an end surface of the housing close to the drive assembly, the mounting cavity includes a first cavity wall, an axial direction of the first cavity wall is parallel to an axial direction of the housing, the sliding member is disposed on the first cavity wall and of a columnar structure, and an axial direction of the sliding member is perpendicular to the first cavity wall.

In one of the embodiments, the movable member is a spherical movable member.

In one of the embodiments, a clamping groove is disposed on the crimping member, and when the crimping member is sleeved on the fixing member, the clamping groove is clamped to the fixing member to restrict relative rotation between the fixing member and the crimping member.

In one of the embodiments, the housing is provided with at least two sliding members, the guide member is provided with at least two guide slots, and the at least two guide slots are disposed in one-to-one correspondence with the at least two sliding members.

### BRIEF DESCRIPTION OF THE DRAWINGS

In order to more clearly illustrate the technical solutions in the embodiments of the present disclosure or the traditional technology, the following will briefly introduce the drawings that need to be used in the description of the embodiments or the traditional technology. Apparently, the drawings in the following description are only embodiments of the present disclosure. For those of ordinary skill in the art, other drawings can also be obtained according to the disclosed drawings without creative efforts.
FIG. 1 is a cross-sectional view illustrating an interventional ultrasound imaging device in a separated state according to some embodiments of the present disclosure.
FIG. 2 is a schematic diagram illustrating a structure of a pressing portion pressed onto a movable member according to some embodiments of the present disclosure.
FIG. 3 is a schematic diagram illustrating a structure of a crimping member according to some embodiments of the present disclosure.
FIG. 4 is a schematic diagram illustrating a structure of a guide member according to some embodiments of the present disclosure.
FIG. 5 is a schematic diagram illustrating a structure of clamping between a crimping member and a fixing member according to some embodiments of the present disclosure.
FIG. 6 is a schematic diagram illustrating a structure of a sliding member in a first docking position according to some embodiments of the present disclosure.
FIG. 7 is a schematic diagram illustrating a structure of a pressing portion separated from a movable member when a sliding member is at a first docking position according to some embodiments of the present disclosure.
FIG. 8 is a schematic diagram illustrating a structure of a sliding member at a second docking position according to some embodiments of the present disclosure.
FIG. 9 is a schematic diagram illustrating a structure of a positional relationship between an abutment portion and a movable member when a sliding member is at a second docking position according to some embodiments of the present disclosure.
FIG. 10 is a schematic diagram illustrating a structure of a sliding member at a third docking position according to some embodiments of the present disclosure.
FIG. 11 is a schematic diagram illustrating a structure of a pressing portion abutting against a movable member when a sliding member is at a third docking position according to some embodiments of the present disclosure.
FIG. 12 is a schematic diagram illustrating a structure of a sliding member in a fourth docking position according to some embodiments of the present disclosure.

### DETAILED DESCRIPTION OF THE EMBODIMENTS

The following will clearly and completely describe the technical solutions in the embodiments of the present disclosure with reference to the accompanying drawings in the embodiments of the present disclosure. Apparently, the described embodiments are only a part of the embodiments of the present disclosure, not all of them. Based on the embodiments in the present disclosure, all other embodiments obtained by those of ordinary skill in the art without creative efforts shall fall within the protection scope of the present disclosure.

In the description of the present disclosure, it should be understood that the orientation or position relationship indicated by the terms "center", "longitudinal", "transverse", "length", "width", "thickness", "upper", "lower", "front", "back", "left", "right", "vertical", "horizontal", "top", "bottom", "inner", "outer", "clockwise", "counterclockwise", "axial", "radial", "circumferential", etc. are based on the orientation or position relationship shown in the accompanying drawings and are merely intended to facilitate the description of the present disclosure and simplify the description, rather than indicating or implying that the indicated device or element should have a particular orientation or be constructed and operated in a particular orientation, and therefore are not to be interpreted as limiting the present disclosure.

In addition, if these terms "first", "second" appear, these terms are only used for descriptive purposes and cannot be understood as indicating or implying relative importance or implicitly indicating the number of indicated technical features. Thus, the features defined with "first" and "second" may explicitly or implicitly include at least one of the features. In the description of the present disclosure, if the term "plurality" appears, the meaning of "plurality" is at least two, such as two, three, etc., unless otherwise clearly and specifically defined.

In the present disclosure, unless otherwise clearly specified and limited, if the terms "mounted", "connected", "coupling", "fixed" etc. appear, these terms should be understood in a broad sense. For example, it can be a fixed connection, a detachable connection, or an integral connection. It can be a mechanical connection or an electrical connection. It can be a direct connection or an indirect connection through an intermediate medium, and it can be the internal communication of two elements or the interaction relationship between two elements, unless otherwise clearly limited. For those of ordinary skill in the art, the specific meanings of the above terms in the present disclosure can be understood according to specific situations.

In the present disclosure, unless otherwise clearly specified and limited, if there is a description such as "a first feature is on or under a second feature", its meaning may be that the first and second features are in direct contact, or the first and second features are in indirect contact through an intermediate medium. Moreover, "on", "above" and "over" the first feature on the second feature may mean that the first feature is directly above or obliquely above the second feature, or simply means that the horizontal height of the first feature is higher than that of the second feature. "Under", "below" and "beneath" the first feature on the second feature may mean that the first feature is directly below or diagonally below the second feature, or simply means that the horizontal height of the first feature is lower than that of the second feature.

It should be noted that if an element is referred to as being "fixed to" or "disposed on" another element, it may be directly on the other element or there may be an intermediate element. If an element is considered to be "connected" to another element, it may be directly connected to the other element or there may be an intermediate element at the same time. If present, the terms "vertical", "horizontal", "upper", "lower", "left", "right" and similar expressions used in the present disclosure are only for illustrative purposes and do not represent the only embodiment.

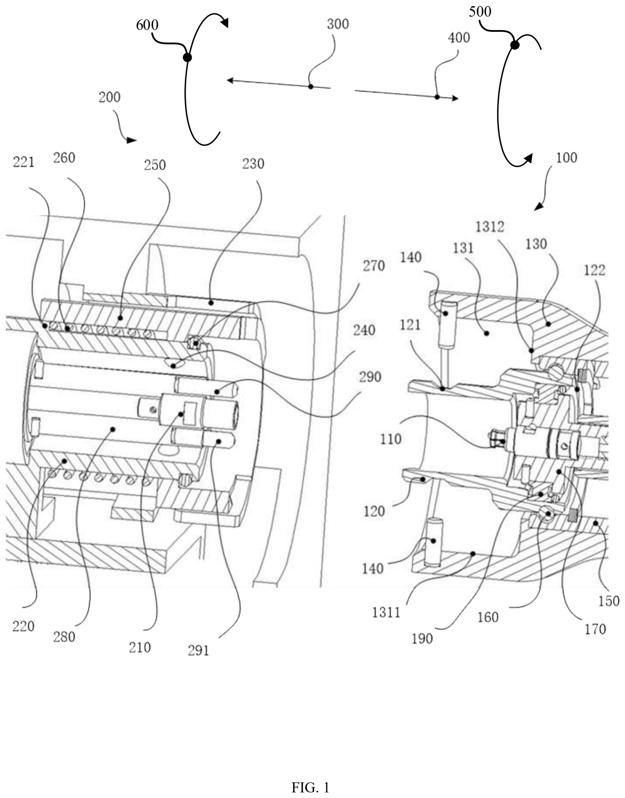

An interventional ultrasound imaging device is provided in the present disclosure. As shown in FIGS. 1 to 12, the interventional ultrasound imaging device includes: an ultrasound probe 100 and a drive assembly 200. The ultrasound probe 100 includes a first connector 110, an inner sheath 120 and a housing 130 sequentially sleeved from inside to outside. The housing 130 is provided with a sliding member 140. The drive assembly 200 includes a second connector 210, a fixing member 220 and a guide member 230 sequentially sleeved from inside to outside. The guide member 230 is provided with a guide slot 234. The interventional ultrasound imaging device has a separated state and a locked state. When the interventional ultrasound imaging device is in the separated state, the first connector 110 is separated from the second connector 210. When the interventional ultrasound imaging device is in the locked state, the first connector 110 is electrically connected to the second connector 210, and the inner sheath 120 extends into the fixing member 220 partially and is connected to the fixing member 220. The sliding member 140 is configured to slide along the guide slot 234 to switch the interventional ultrasound imaging device between the separated state and the locked state. The ultrasound probe 100 further includes a transducer (i.e., an ultrasonic transducer), which is fixedly mounted at an end of a first rotating shaft 170 away from the drive assembly 200, and is located inside the inner sheath 120 and on a side of the first connector 110 facing a front end of the ultrasound probe 100. An axis of the transducer coincides with an axis of the first rotating shaft 170. The transducer is configured to transmit and receive ultrasonic imaging signals.

It should be noted that an electrical connection enables the transmission of electrical signals between mutually mated conductive components. Specifically, when the first connector 110 is electrically connected to the second connector 210, ultrasonic imaging signals and drive control signals are transmitted between the ultrasound probe 100 and the drive assembly 200.

In the above interventional ultrasound imaging device, the inner sheath 120 of the ultrasound probe 100 is sleeved on the first connector 110, the housing 130 is sleeved on the inner sheath 120, the fixing member 220 of the drive assembly 200 is sleeved on the second connector 210, and the guide member 230 is sleeved on the fixing member 220. By disposing sliding member 140 on the housing 130 and disposing the guide slot 234 on the guide member 230, when it is necessary to lock the ultrasound probe 100 and the drive assembly 200, i.e., to switch the interventional ultrasound imaging device to the locked state, the ultrasound probe 100 is moved towards the drive assembly 200 to make the sliding member 140 slide along the guide slot 234, so that the first connector 110 and the second connector 210 are electrically connected to each other, and the inner sheath 120 extends into the fixing member 220 partially, so that the inner sheath 120 is connected to the fixing member 220. At this time, the drive assembly 200 can drive the ultrasound probe 100 to perform linear motion or rotational motion. When it is necessary to separate the ultrasound probe 100 from the drive assembly 200, the first connector 110 and the second connector 210, as well as the inner sheath 120 and the fixing member 220 can be separated from each other simply by reversely sliding the sliding member 140 in the guide slots 234, and then the ultrasound probe 100 can be moved away from the drive assembly 200 by reversely pulling the ultrasound probe 100, thereby switching the interventional ultrasound imaging device to the separated state. Compared with traditional interventional ultrasound imaging devices, the interventional ultrasound imaging device of the present disclosure can simultaneously realize rotational motion transmission, linear motion transmission and electrical connection docking, and has more comprehensive functions. Moreover, the ultrasound probe 100 and the drive assembly 200 can be integrally docked, which is more convenient to use.

Specifically, as shown in FIG. 1, an inwardly recessed mounting cavity 131 is disposed on an end surface of the housing 130 close to the drive assembly 200, and the sliding member 140 is disposed on a cavity wall of the mounting cavity 131. In some examples, the sliding member 140 can be disposed on the cavity wall of the mounting cavity 131 through interference fit or glue bonding to be fixedly connected to the housing 130, and the sliding member 140 can also be integrally formed with the cavity wall of the mounting cavity 131.

More specifically, as shown in FIG. 1, the mounting cavity 131 includes a first cavity wall 1311, and an axial direction of the first cavity wall 1311 is parallel to an axial direction of the housing 130. The sliding member 140 is disposed on the first cavity wall 1311. In some examples, the axial direction of the first cavity wall 1311 coincides with the axial direction of the housing 130.

More specifically, in this embodiment, as shown in FIG. 1, the sliding member 140 is of a columnar structure, and an axial direction of the sliding member 140 is perpendicular to the first cavity wall 1311. In other embodiments, the sliding member 140 can be of any structure as long as it is capable of sliding along the guide slot 234.

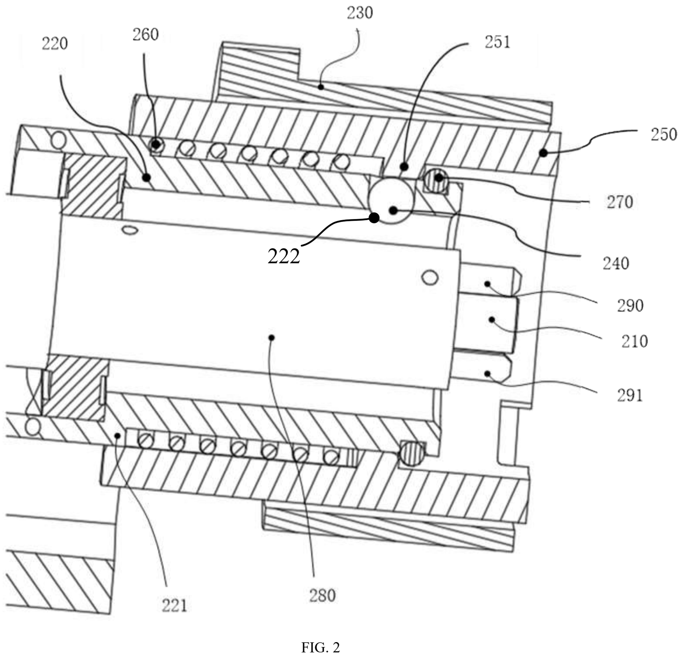

Further, in order to realize a connection between the fixing member 220 and the inner sheath 120, as shown in FIGS. 1 and 2, a through hole 222 is disposed on the fixing member 220, a movable member 240 is movably disposed in the through hole 222, and a limiting groove 121 is disposed on the inner sheath 120. When the device is in the locked state, the movable member 240 is partially located in the through hole 222 and partially located in the limiting groove 121 to connect the fixing member 220 and the inner sheath 120. By disposing the through hole 222 and disposing the movable member 240 in the through hole 222, when the movable member 240 is partially located in the through hole 222 and partially located in the limiting groove 121, a relative position between the fixing member 220 and the inner sheath 120 is fixed, so that the fixing member 220 and the inner sheath 120 are connected to each other. In some examples, the movable member 240 can be a spherical movable member, such as a steel ball.

Specifically, as shown in FIGS. 1 to 3, the drive assembly 200 includes a crimping member 250, the crimping member 250 is disposed between the guide member 230 and the fixing member 220, and the crimping member 250 is configured to restrict the movable member 240 from being disengaged from the through hole 222. By disposing the crimping member 250 between the guide member 230 and the fixing member 220, the crimping member 250 can abut against the movable member 240, thereby restricting the movable member 240 from being disengaged from the through hole 222.

More specifically, the crimping member 250 is cylindrical, and the fixing member 220, the crimping member 250 and the guide member 230 are sequentially sleeved from inside to outside.

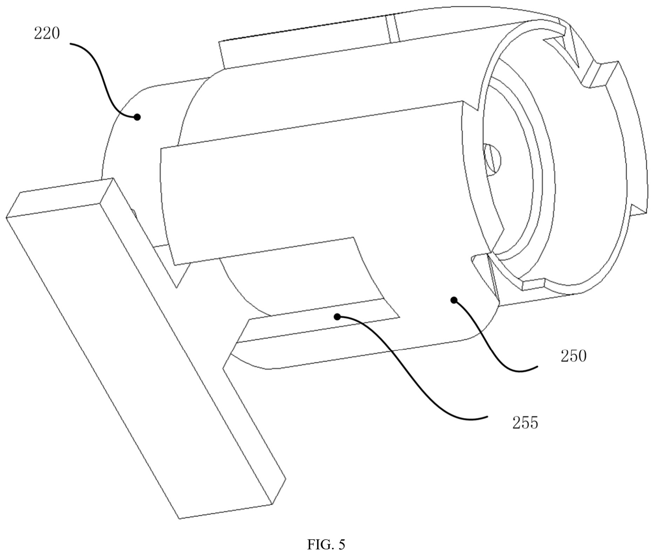

Specifically, as shown in FIG. 5, a clamping groove 255 is disposed on the crimping member 250, and when the crimping member 250 is sleeved on the fixing member 220, the clamping groove 255 is clamped to the fixing member 220 to restrict relative rotation between the fixing member 220 and the crimping member 250.

More specifically, as shown in FIG. 5, the clamping groove 255 extends along its own axial direction, so that the crimping member 250 can move axially relative to the fixing member 220.

Specifically, as shown in FIGS. 1 to 3, the through hole 222 has a large end and a small end, the large end faces the crimping member 250, and the small end faces the second connector 210. A pressing portion 251 is disposed on an inner cavity wall of the crimping member 250, and the pressing portion 251 is attached onto a side of the movable member 240 away from the second connector 210, so that the movable member 240 is partially disposed in the through hole 222 and partially disposed in the limiting groove 121. The crimping member 250 is sleeved on the fixing member 220, and the pressing portion 251 is disposed on the inner cavity wall of the crimping member 250. By attaching the pressing portion 251 onto the side of the movable member 240 away from the second connector 210, to press the movable member 240, the fixing member 220 and the inner sheath 120 are connected to each other through the movable member 240.

Further, as shown in FIGS. 1 to 9, the ultrasound probe 100 is capable of approaching the drive assembly 200 in a first direction 300 to dock the ultrasound probe 100 with the drive assembly 200, and the guide slot 234 includes a first sliding portion 231 parallel to the first direction 300. An abutment groove 252, which is inwardly recessed, is disposed at an end of the crimping member 250 close to the ultrasound probe 100, and a length of the first sliding portion 231 in the first direction 300 is greater than a length of the abutment groove 252 in the first direction 300. The sliding member 140 is capable of extending through the first sliding portion 231 and the abutment groove 252 simultaneously and abuts against a groove wall of the abutment groove 252, and the sliding member 140 is capable of sliding along the first sliding portion 231 to push the crimping member 250 to move in the first direction 300, so that the pressing portion 251 is separated from the movable member 240.

More specifically, the first direction 300 is parallel to an axial direction of the ultrasound probe 100.

It should be noted that the first direction 300 in which the ultrasound probe 100 approaches the drive assembly 200 is the direction in which the ultrasound probe 100 moves axially towards the drive assembly 200, i.e., a horizontal left direction as shown in FIG. 1.

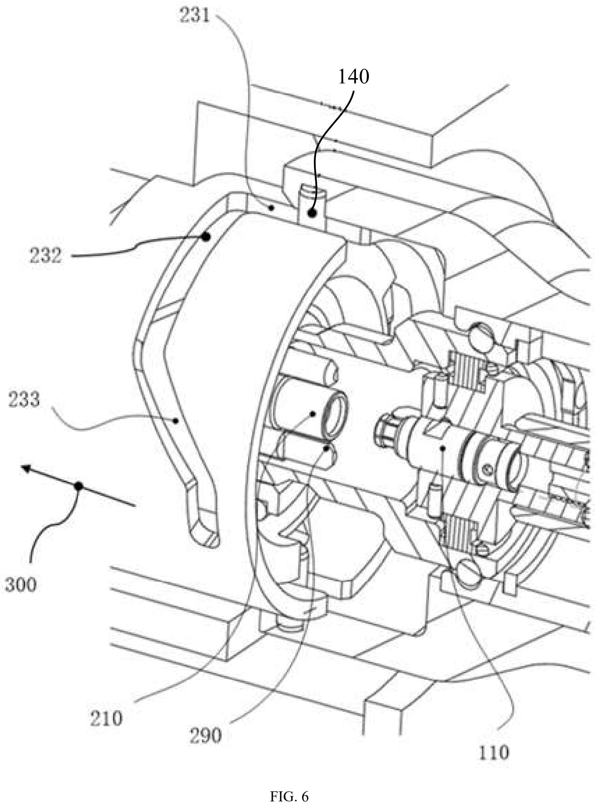

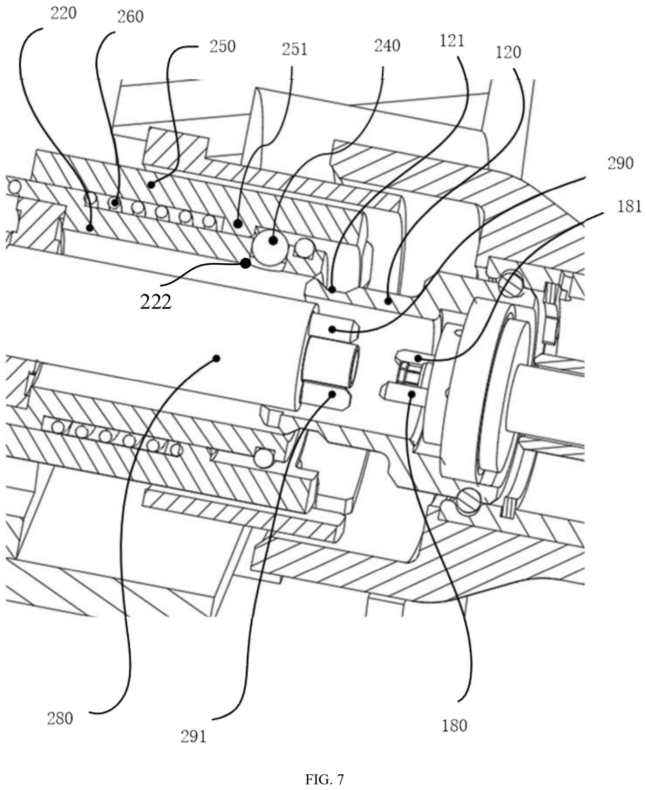

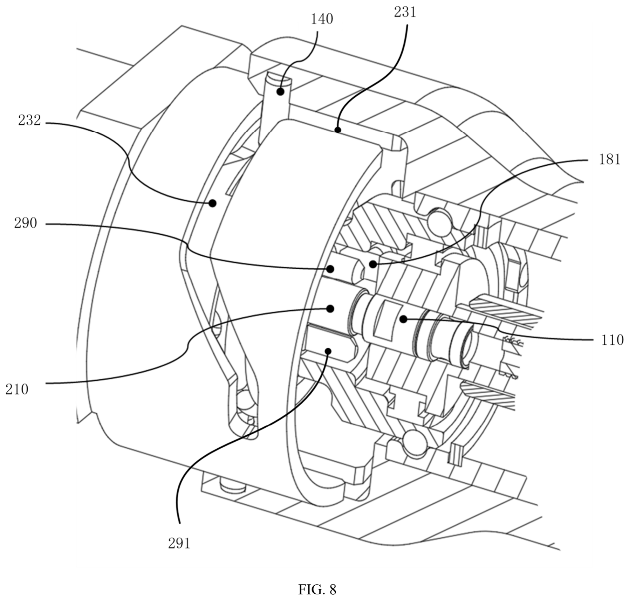

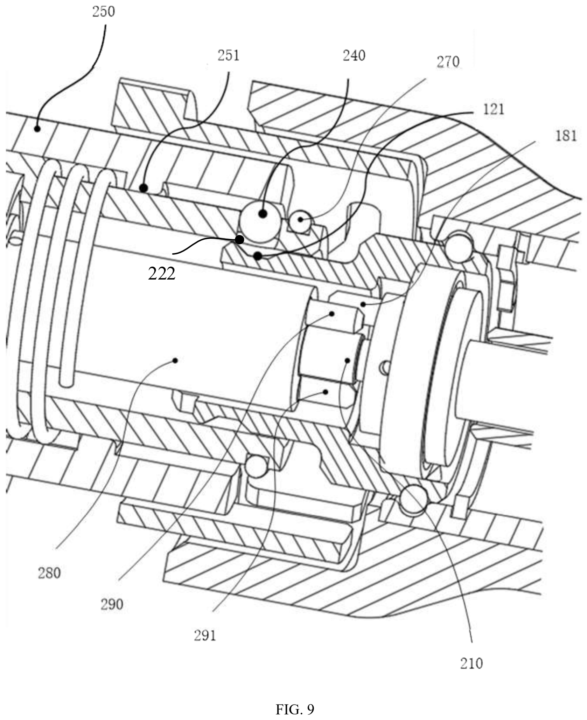

When it is necessary to dock the ultrasound probe 100 with the drive assembly 200, the ultrasound probe 100 is driven to approach the drive assembly 200 in the first direction 300, so that the sliding member 140 on the ultrasound probe 100 extends through the first sliding portion 231 and the abutment groove 252 simultaneously. The sliding member 140 slides along the first sliding portion 231 to abut against the groove wall of the abutment groove 252. The sliding member 140 continues to slide along the first sliding portion 231 to push the crimping member 250 to move in the first direction 300 relative to the fixing member 220, thereby making the pressing portion 251 of the crimping member 250 move in the first direction 300 to separate from the movable member 240. At this time, the movable member 240 is not subjected to external force and is in a free state (as shown in FIGS. 6 and 7). The sliding member 140 continues to slide along the first sliding portion 231. At this time, the inner sheath 120 extend into the cavity of the fixing member 220, and the through hole 222 on the fixing member 220 is aligned with the limiting groove 121 on the inner sheath 120 (as shown in FIGS. 8 and 9).

It should be noted that the through hole 222 on the fixing member 220 being aligned with the limiting groove 121 on the inner sheath 120 means that the through hole 222 and the limiting groove 121 are aligned in a radial direction of the drive assembly 200, i.e., an arrangement direction of the through hole 222 and the limiting groove 121 is perpendicular to the first direction 300.

It should be noted that the length of the first sliding portion 231 in the first direction 300 is greater than the length of the abutment groove 252 in the first direction 300. During sliding of the sliding member 140 along the first sliding portion 231, the sliding member 140 has a first position and a second position. When the sliding member 140 slides to the first position, the pressing portion 251 of the crimping member 250 is separated from the movable member 240 (as shown in FIGS. 6 and 7), and there is a certain gap between the through hole 222 on the fixing member 220 and a left end surface of the inner sheath 120 (as shown in FIG. 7, the left end surface). At this time, the through hole 222 on the fixing member 220 is not aligned with the limiting groove 121 on the inner sheath 120. When the sliding member 140 slides to the second position, the through hole 222 on the fixing member 220 is aligned with the limiting groove 121 on the inner sheath 120 (as shown in FIGS. 8 and 9), and the first connector 110 of the ultrasound probe 100 is electrically connected to the second connector 210 of the drive assembly 200.

The pressing portion 251 has a first side wall (i.e., a right side wall of the pressing portion 251 as shown in FIG. 2) close to the ultrasound probe 100, and the through hole 222 of the fixing member 220 has a second side wall, which is a side wall section away from the ultrasound probe 100 (i.e., a left section of the side wall of the through hole 222 as shown in FIG. 2). The first position is a position (as shown in FIG. 7) where the first side wall and the second side wall are aligned in the radial direction of the drive assembly 200. The second position is a position where the sliding member 140 is at an end of the first sliding portion 231.

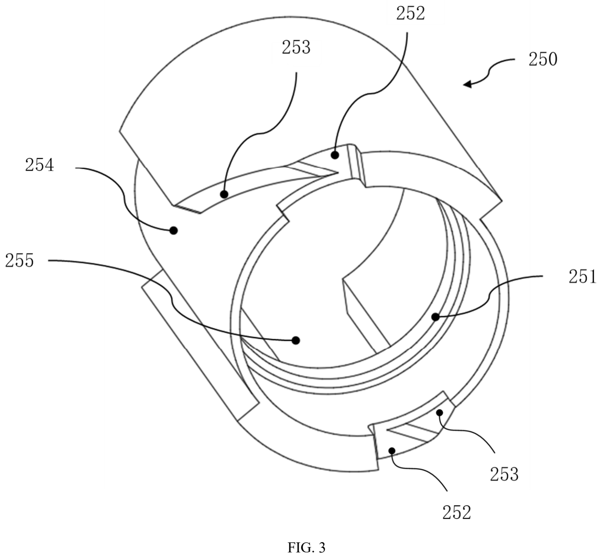

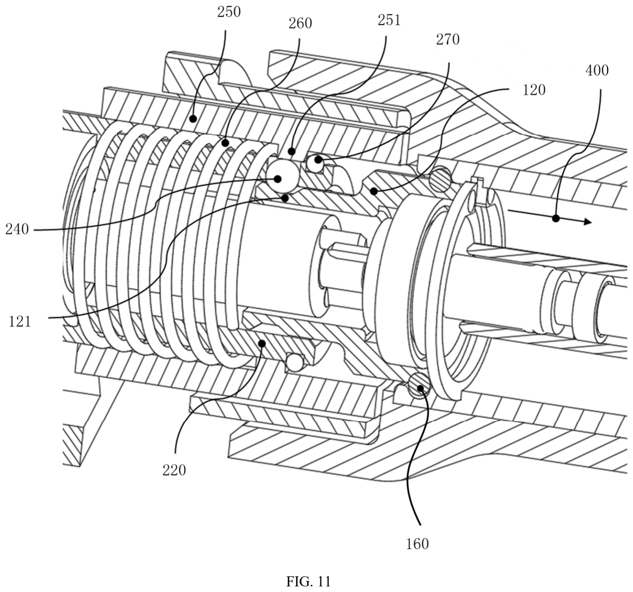

When the sliding member 140 is at the second position, i.e., after the through hole 222 on the fixing member 220 is aligned with the limiting groove 121 on the inner sheath 120, in order to connect the fixing member 220 and the inner sheath 120 by using the movable member 240, as shown in FIGS. 3 to 11, the drive assembly 200 of the present disclosure further includes a reset member 260. A protrusion 221 is disposed on the fixing member 220, the reset member 260 is sleeved on the fixing member 220, an end of the reset member 260 abuts against the pressing portion 251, and another end of the reset member 260 abuts against the protrusion 221. The reset member 260 is capable of pushing the crimping member 250 to move in a second direction 400 through its own elastic force. The second direction 400 is opposite to the first direction 300. The reset member 260 is sleeved on the fixing member 220, and the pressing portion 251 is pushed to move in the second direction 400 by the reset member 260, so that the pressing portion 251 presses the movable member 240, and the movable member 240 is partially disposed in the through hole 222 and partially disposed in the limiting groove 121, thereby connecting the fixing member 220 and the inner sheath 120 to each other (as shown in FIG. 11).

It should be noted that the first direction 300 in which the ultrasound probe 100 approaches the drive assembly 200 is a direction in which the ultrasound probe 100 moves axially towards the drive assembly 200. If the second direction 400 is a direction opposite to the first direction 300, the second direction 400 is a direction in which the ultrasound probe 100 moves axially away from the drive assembly 200, i.e., a horizontal right direction as shown in FIG. 1.

It should be noted that the first direction 300 is parallel to the axial direction of the ultrasound probe 100, and the second direction 400 is a direction opposite to the first direction 300, so the second direction 400 is also parallel to the axial direction of the ultrasound probe 100.

Specifically, the reset member 260 can be an elastic reset member, such as a spring.

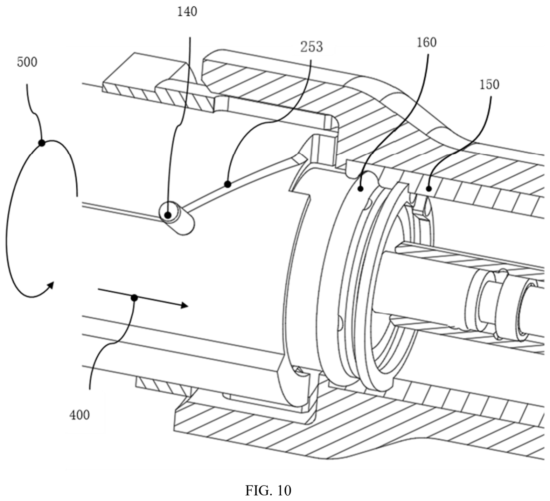

Specifically, as shown in FIGS. 3 to 11, the guide slot 234 includes a second sliding portion 232 communicating with the first sliding portion 231, and the second sliding portion 232 extends in a circumferential direction of the guide member 230. An inclined surface groove 253 communicating with the abutment groove 252 is disposed on an outer peripheral surface of the crimping member 250, and a groove wall of the inclined surface groove 253 extends from a groove wall of the abutment groove 252 in a direction away from the ultrasound probe 100. The sliding member 140 is capable of extending through the second sliding portion 232 and the inclined surface groove 253 simultaneously and sliding along the second sliding portion 232, so that the reset member 260 pushes the pressing portion 251 to move in the second direction 400. The second sliding portion 232 is connected to the first sliding portion 231, and the inclined surface groove 253 is disposed on the crimping member 250 corresponding to the second sliding portion 232. When the sliding member 140 is at the second position, the sliding member 140 slides from the first sliding portion 231 into the second sliding portion 232 and extends through the second sliding portion 232 and the inclined surface groove 253 simultaneously. The sliding member 140 continues to slide along the second sliding portion 232. Since the second sliding portion 232 extends in the circumferential direction of the guide member 230, and the groove wall of the inclined surface groove 253 extends from the groove wall of the abutment groove 252 in a direction away from the ultrasound probe 100. When the sliding member 140 slides into the second sliding portion 232 to start to slide along the second sliding portion 232, under an elastic force of the reset member 260, the crimping member 250 moves in the second direction 400, so that the pressing portion 251 of the crimping member 250 presses the movable member 240, so that the movable member 240 is partially disposed in the through hole 222 and partially disposed in the limiting groove 121 (as shown in FIGS. 10 and 11). During movement of the crimping member 250 in the second direction 400, the sliding member 140 abuts against the groove wall of the inclined surface groove 253, thereby limiting a movement distance of the crimping member 250 in the second direction 400.

It should be noted that the sliding member 140 has a third position. The third position is a position where the sliding member 140 is at an end of the second sliding portion 232. When the sliding member 140 is at the third position, the movable member 240 is partially disposed in the through hole 222 and partially disposed in the limiting groove 121, so that the inner sheath 120 and the fixing member 220 are in a connected state.

Specifically, in this embodiment, as shown in FIGS. 1 to 11, the second sliding portion 232 extends in the circumferential direction of the guide member 230. When the ultrasound probe 100 is docked with the drive assembly 200, i.e., when the interventional ultrasound imaging device is adjusted to be in the locked state, after the sliding member 140 slides into the second sliding portion 232, the sliding member 140 slides in a third direction 500 to connect the inner sheath 120 and the fixing member 220 to each other. The third direction 500 is a counterclockwise rotation direction around the first direction 300. In order to separate the ultrasound probe 100 from the drive assembly 200, i.e., adjust the interventional ultrasound imaging device to be in the separated state, it is only necessary to slide the sliding member 140 in the second sliding portion 232 in a fourth direction 600. The fourth direction 600 is a clockwise rotation direction around the first direction 300.

It can be understood that in this embodiment, the third direction 500 is a counterclockwise rotation direction around the first direction 300, and the fourth direction 600 is a clockwise rotation direction around the first direction 300. In other embodiments, according to the adjustment of the extension direction of the second sliding portion 232, the third direction 500 can be a clockwise rotation direction around the first direction 300, and the fourth direction 600 can be a counterclockwise rotation direction around the first direction 300.

Specifically, the first projection plane is a projection plane of the crimping member 250 that is perpendicular to the axial direction (i.e., the first direction 300 or the second direction 400) of the crimping member. A projection of the inclined surface groove 253 in the axial direction of the crimping member 250 on the first projection plane forms a first arc, and an arc length of the first arc is less than an arc length of the second sliding portion 232. By defining the arc lengths of the second sliding portion 232 and the first arc, it is ensured that when the sliding member 140 slides in the third direction 500, the crimping member 250 can move in the second direction 400 under the action of the reset member 260 and press the movable member 240.

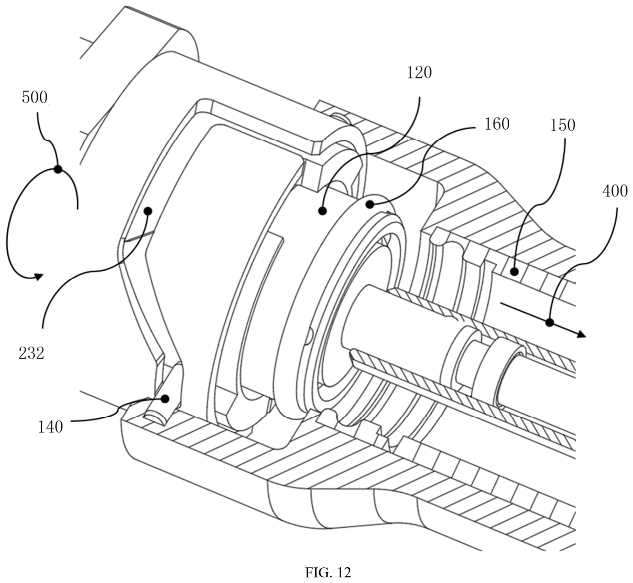

Further, as shown in FIGS. 1 and 12, the ultrasound probe 100 includes an outer sheath 150 and a damping member 160. The outer sheath 150 is connected to the housing 130, the damping member 160 is sleeved on the inner sheath 120, and the outer sheath 150 is connected to the inner sheath 120 through the damping member 160. The outer sheath 150 is connected to the housing 130, and the damping member 160 is sleeved on the inner sheath 120, i.e., the ultrasound probe 100 is sequentially sleeved from inside to outside with: the first connector 110, the inner sheath 120, the damping member 160, the outer sheath 150, and the housing 130. The inner sheath 120 and the related structures inside the inner sheath 120 are protected by the outer sheath 150. The damping member 160 is provided to connect the outer sheath 150 and the inner sheath 120 to each other.

Specifically, in this embodiment, the damping member 160 is sleeved on the inner sheath 120, and the damping member 160 is in interference fit with the inner sheath 120. In other embodiments, the damping member 160 can be secured to the inner sheath 120 through other connection structures.

Further, when the ultrasound probe 100 is docked with the drive assembly 200, i.e., the first connector 110 is electrically connected to the second connector 210, and the inner sheath 120 extends into the fixing member 220 partially and is connected thereto, i.e., the sliding member 140 slides to the end of the second sliding portion 232, the sliding member 140 is further adjusted to slide, and the outer sheath 150 is separated from the damping member 160, to separate of the outer sheath 150 from the inner sheath 120, and prevent the outer sheath 150 from affecting the axial movement of the inner sheath 120 and the first connector 110.

Specifically, as shown in FIGS. 1, 3, 4 and 12, a recessed portion 254 communicating with the inclined surface groove 253 is disposed on the outer peripheral surface of the crimping member 250. The guide slot 234 includes a third sliding portion 233 communicating with the second sliding portion 232, and the third sliding portion 233 extends from an end of the second sliding portion 232 away from the first sliding portion 231 in a direction close to the ultrasound probe 100. The sliding member 140 extends through the third sliding portion 233 and the recessed portion 254, and slides in a direction away from the second sliding portion 232 to separate the damping member 160 from the outer sheath 150. The sliding member 140 extends through the third sliding portion 233 and the recessed portion 254 and slides in a direction close to the second sliding portion 232 to connect the damping member 160 to the outer sheath 150.

By providing the third sliding portion 233 communicating with the second sliding portion 232 and extending the third sliding portion 233 towards the ultrasound probe 100, when the sliding member 140 is at the third position, i.e., the sliding member 140 is at the end of the second sliding portion 232, the sliding member 140 slides from the second sliding portion 232 into the third sliding portion 233, and the sliding member 140 extends through the third sliding portion 233 and the recessed portion 254 simultaneously. The sliding member 140 continues to slide along the third sliding portion 233 in a direction away from the second sliding portion 232 (i.e., as shown in FIG. 12, the sliding member 140 slides downward along the third sliding portion 233), and when sliding to an end position, the outer sheath 150 moves a predetermined movement distance in the second direction 400. At this time, the inner sheath 120 remains stationary, and the outer sheath 150 is separated from the damping member 160, thereby realizing the separation of the inner sheath 120 from the outer sheath 150. The predetermined movement distance can be the axial displacement of the outer sheath 150 from being sleeved on the damping member 160 to being separated from the damping member 160. When it is necessary to realize the connection between the inner sheath 120 and the outer sheath 150, the sliding member 140 extends through the third sliding portion 233 and the recessed portion 254 simultaneously, slides along the third sliding portion 233 in a direction close to the second sliding portion 232 (i.e., as shown in FIG. 12, the sliding member 140 slides upward along the third sliding portion 233), and realizes the connection between the damping member 160 and the outer sheath 150, so that the inner sheath 120 and the outer sheath 150 can be connected to each other.

It should be noted that because the length of the first arc is less than the arc length of the second sliding portion 232, and the inclined surface groove 253 communicates with the recessed portion 254, when the sliding member 140 slides along the second sliding portion 232, there should be a case where the sliding member 140 extends through the second sliding portion 232 and the recessed portion 254 at the same time. When the sliding member 140 extends through the end of the second sliding portion 232 and slides into the third sliding portion 233, the sliding member 140 extends through the third sliding portion 233 and the recessed portion 254 simultaneously.

Further, in order to enable the sliding member 140 to smoothly slide along the third sliding portion 233 to the end of the third sliding portion 233 without affecting the sliding of the sliding member 140, during the sliding process, the relevant dimensions are defined to prevent the sliding member 140 from abutting against the side wall of the recessed portion 254.

Specifically, the second projection plane is a projection plane of the guide member 230 perpendicular to its own axial direction. The projection of the third sliding portion 233 in the axial direction of the guide member 230 on the second projection plane forms a second arc. The projection of the recessed portion 254 in the axial direction of the crimping member 250 on the first projection plane forms a third arc. In order to prevent that the sliding member 140 slides in the third direction 500 from being hindered by the inclined surface groove 253 and the recessed portion 254 disposed on the outer peripheral surface of the crimping member 250, the sum of the arc length of the second sliding portion 232 and the arc length of the second arc should be less than the sum of the arc length of the first arc and the arc length of the third arc.

Specifically, as shown in FIG. 3, in this embodiment, the recessed portion 254 is a recessed surface extending through the outer peripheral surface of the crimping member 250, and the arc length of the third arc of the recessed portion 254 is the arc length of the circumferential surface corresponding to the recessed portion 254.

In other embodiments, the recessed portion 254 can also be in a long groove structure or the like, as long as it can accommodate the end of the sliding member 140 and allow the end of the sliding member 140 to slide. In some examples, the sliding member 140 is a pin.

Further, as shown in FIGS. 1, 4 and 12, the length of the third sliding portion 233 in the first direction 300 is greater than or equal to the predetermined movement distance of the outer sheath 150. By defining the relationship between the length of the third sliding portion 233 in the first direction 300 and the predetermined movement distance of the outer sheath 150, it is ensured that when the sliding member 140 slides along the third sliding portion, the outer sheath 150 can move the predetermined movement distance in the second direction 400.

It should be noted that the length of the third sliding portion 233 in the first direction 300 is the dimension of the third sliding portion 233 along its own axial direction.

Specifically, as shown in FIG. 1, an inwardly recessed mounting cavity 131 is disposed on an end surface of the housing 130 close to the drive assembly 200. The mounting cavity 131 includes a second cavity wall 1312 (i.e., a cavity wall of the mounting cavity 131 away from the drive assembly 200 as shown in FIG. 1) perpendicular to the second direction. The inner sheath 120 has a first end surface 122 (i.e., a right end surface of the inner sheath 120 as shown in FIG. 1) away from the drive assembly 200. The distance between the second cavity wall 1312 and the first end surface 122 in the second direction 400 is the predetermined movement distance of the outer sheath 150.

Further, referring to FIG. 1, the drive assembly 200 further includes a retaining ring 270. The retaining ring 270 is sleeved on the fixing member 220 and disposed on a side of the movable member 240 close to the ultrasound probe 100. When the pressing portion 251 is attached onto the movable member 240, the retaining ring 270 abuts against the pressing portion 251. By disposing the retaining ring 270 on the fixing member 220 and on the side of the movable member 240 close to the ultrasound probe 100, when the interventional ultrasound imaging device is in the locked state, i.e., the movable member 240 is partially disposed in the through hole 222 and partially disposed in the limiting groove 121 of the inner sheath 120 to connect the fixing member 220 and the inner sheath 120 to each other, the retaining ring 270 abuts against the pressing portion 251, which not only restricts the crimping member 250 from being disengaged from the fixing member 220, but also further limits the relative position between the crimping member 250 and the movable member 240, so that the pressing portion 251 of the crimping member 250 can press the movable member 240.

Further, referring to FIGS. 1, 2, 6 and 7, the ultrasound probe 100 further includes a first rotating shaft 170 extending through the inner sheath 120, the first connector 110 is mounted on the first rotating shaft 170, and a first transmission member 180 is mounted on the first rotating shaft 170. The drive assembly 200 further includes a second rotating shaft 280 extending through the fixing member 220, the second connector 210 is mounted on the second rotating shaft 280, and a second transmission member 290 is mounted on the second rotating shaft 280. When the interventional ultrasound imaging device is in the locked state, the second transmission member 290 is configured to contact the first transmission member 180, ensuring the drive assembly 200 drives the ultrasound probe 100 to rotate. The contact may include abutment and angular contact. By providing the first transmission member 180 and the second transmission member 290, when the interventional ultrasound imaging device is in the locked state, the first transmission member 180 and the second transmission member 290 are adjusted to abut against each other, thereby transmitting the power of the drive assembly 200 to the ultrasound probe 100.

After the ultrasound probe 100 is docked with the drive assembly 200 and is in the locked state, the first transmission member 180 and the second transmission member 290 have two positional relationships.

In some embodiments, the first transmission member 180 and the second transmission member 290 are in a direct abutment state. At this time, the first transmission member 180 can be driven to rotate by rotating the second transmission member 290, thereby driving the ultrasound probe 100 to rotate.

In some embodiments, there is a gap between the first transmission member 180 and the second transmission member 290. At this time, it is necessary to rotate the second transmission member 290 to make the second transmission member 290 abut against the first transmission member 180, and then drive the first transmission member 180 to rotate through the second transmission member 290.

Specifically, as shown in FIGS. 1, 2, 6 and 7, the first transmission member 180 includes a first abutment plane 181, and the second transmission member 290 includes a second abutment plane 291. When the interventional ultrasound imaging device is in the locked state, the first abutment plane 181 is configured to be attached onto the second abutment plane 291. By adjusting the arrangement to make the first abutment plane 181 to be attached onto the second abutment plane 291, the contact area between the first transmission member 180 and the second transmission member 290 is increased, and the stability of power transmission is improved.

More specifically, the first transmission member 180 and the second transmission member 290 are semicylinders obtained by cutting a cylinder in half along its own axial direction, and the cut surface forms the first abutment plane 181 or the second abutment plane 291.

Specifically, a plurality of first transmission members 180 and a plurality of second transmission members 290 are provided, and the plurality of first transmission members 180 are in one-to-one correspondence with the plurality of second transmission members 290.

In this embodiment, as shown in FIGS. 1, 2, 6 and 7, two first transmission members 180 and two second transmission members 290 are provided. The two first transmission members 180 are arranged in the radial direction of the first connector 110, and the two first abutment planes 181 are oriented in opposite directions. The two second transmission members 290 are arranged in the radial direction of the second connector 210, and the two second abutment planes 291 are oriented in opposite directions.

Specifically, the ultrasound probe 100 further includes a bearing 190, and the first rotating shaft 170 is rotatably connected to the inner sheath 120 through the bearing 190. The bearing 190 is arranged between the outer peripheral wall of the first rotating shaft 170 and the inner peripheral wall of the inner sheath 120 to reduce the frictional resistance when the first rotating shaft 170 rotates.

Further, a plurality of sliding members 140 are provided in the present disclosure, which are arranged around the circumferential direction of the housing 130. A plurality of guide slots 234 are disposed on the guide member 230, and the plurality of guide slots 234 are in one-to-one correspondence with the plurality of sliding members 140.

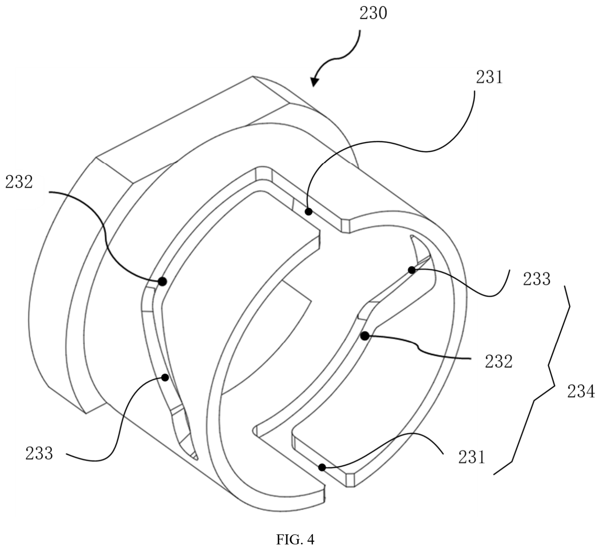

In this embodiment, as shown in FIGS. 1 and 4, the housing 130 is provided with at least two sliding members 140, the guide member 230 is provided with at least two the guide slots 234, and the at least two guide slots 234 are disposed in one-to-one correspondence with the at least two sliding members 140. The cooperation of the plurality of pairs of the sliding member and the guide slot can increase the reliability of the connection between the housing 130 and the guide member 230. As shown in FIGS. 1 and 4, two sliding members 140 are provided, and both sliding members 140 are disposed on the inner wall of the housing 130, and the two sliding members 140 are symmetrically disposed in the axial direction of the ultrasound probe 100. Two guide slots 234 corresponding to the two sliding members 140 one by one are disposed on the guide member 230.

In other embodiments, the number of sliding members 140 can be set to four, six or even eight according to actual operation needs.

Further, the sliding member 140 extends through the guide slot 234 and simultaneously extends into the abutment groove 252, the inclined surface groove 253 or the recessed portion 254 of the crimping member 250. Accordingly, the number of the abutment grooves 252, the inclined surface grooves 253 and the recessed portions 254 on the crimping member 250 is in one-to-one correspondence with the number of the sliding members 140. For example, if two sliding members 140 are provided, two abutment grooves 252, two inclined surface grooves 253 and two recessed portions 254 are also provided.

Further, referring to FIG. 3, in this embodiment, the crimping member 250 is cylindrical, a pressing portion 251 is disposed on the inner peripheral surface of the crimping member 250, and the pressing portion 251 is annular. The abutment groove 252 is disposed on an end surface of the crimping member 250 close to the ultrasound probe 100, and the inclined surface groove 253 and the recessed portion 254 communicating with the inclined surface groove 253 are disposed on the outer peripheral surface of the crimping member 250, and the inclined surface groove 253 communicates with the abutment groove 252.

Specifically, as shown in FIG. 3, the recessed portion 254 extends through the outer peripheral surface of the crimping member 250.

Specifically, the inclined surface groove 253 is a groove that does not extend through the outer peripheral surface of the crimping member 250.

Further, referring to FIG. 4, in this embodiment, the guide slot 234 is disposed on the guide member 230. The guide slot 234 includes a first sliding portion 231, a second sliding portion 232, and a third sliding portion 233. The first sliding portion 231 extends in the first direction 300, the second sliding portion 232 extends in the circumferential direction of the guide member 230 from an end of the first sliding portion 231, and the third sliding portion 233 extends from an end of the second sliding portion 232 away from the first sliding portion 231 in a direction close to the ultrasound probe 100.

Specifically, as shown in FIG. 4, the third projection plane is a projection plane parallel to the axial direction of the guide member 230. A projection of the second sliding portion 232 in the direction perpendicular to the third projection plane (i.e., the radial direction of the guide member 230) on the third projection plane forms a first projection straight line, which is perpendicular to the first direction 300.

Specifically, as shown in FIG. 4, the third sliding portion 233 extends in the circumferential direction of the guide member 230 away from the first sliding portion 231 and towards the ultrasound probe 100.

More specifically, as shown in FIG. 4, the third sliding portion 233 includes a first region and a second region connected to each other. The first region is connected to the end of the second sliding portion 232, and the first region is inclined. The first region extends from the end of the second sliding portion 232 towards the ultrasound probe 100, i.e., the distance between the first region and the end surface of the guide member 230 close to the ultrasound probe 100 (the right end surface of the guide member 230 as shown in FIG. 4) gradually decreases. The projection of the second region in the direction perpendicular to the third projection plane (i.e., the radial direction of the guide member 230) on the third projection plane forms a second projection straight line, which is perpendicular to the first direction 300, i.e., the first projection straight line is parallel to the second projection straight line, i.e., the second region is parallel to the second sliding portion 232.

More specifically, as shown in FIG. 4, the first region extends from the end of the second sliding portion 232 away from the first sliding portion 231 towards the ultrasound probe 100, and in the circumferential direction of the guide member 230, the distance between the first region and the first sliding portion 231 gradually increases. That is, the distance from the end of the first region close to the second sliding portion 232 to the first sliding portion 231 is less than the distance from the end of the first region away from the second sliding portion 232 to the first sliding portion 231. That is, the first region extends in the circumferential direction of the guide member 230 away from the first sliding portion 231.

In summary, the present disclosure also provides a docking process between the ultrasound probe 100 and the drive assembly 200.
1. Drive the ultrasound probe 100 to approach the drive assembly 200 in the first direction 300. At this time, the sliding member 140 on the ultrasound probe 100 extends through the first sliding portion 231 and the abutment groove 252 simultaneously. When the sliding member 140 slides along the first sliding portion 231 and is at the first position, as shown in FIGS. 6 and 7, the pressing portion 251 is separated from the movable member 240, and the movable member 240 is in a free state.
2. The sliding member 140 continues to push the crimping member 250 to slide along the first sliding portion 231 of the guide slot 234 and is at the second position. As shown in FIGS. 8 and 9, at this time, the through hole 222 on the fixing member 220 is aligned with the limiting groove 121 on the inner sheath 120, the first connector 110 is docked with the second connector 210, the first abutment plane 181 of the first transmission member 180 and the second abutment plane 291 of the second transmission member 290 are configured to be attachable, and at this time, the drive assembly 200 and the ultrasound probe 100 can perform rotational transmission.
3. The sliding member 140 slides into the second sliding portion 232 of the guide slot 234 and slides in the third direction 500 to be at the third position. As shown in FIGS. 10 and 11, under the elastic force of the reset member 260, the crimping member 250 moves in the second direction 400, and the pressing portion 251 abuts against the retaining ring 270. At this time, the pressing portion 251 presses the movable member 240, so that the movable member 240 is partially disposed in the through hole 222 and partially disposed in the limiting groove 121 to realize the connection between the inner sheath 120 and the fixing member 220. At this time, the drive assembly 200 and the ultrasound probe 100 can perform linear transmission.
4. As shown in FIG. 12, the sliding member 140 slides from the second sliding portion 232 into the third sliding portion 233 and slides in the third direction 500 to reach the end of the third sliding portion 233. At this time, the inner sheath 120 remains stationary, the outer sheath 150 is separated from the damping member 160, and the outer sheath 150 moves a predetermined movement distance in the second direction 400, thereby realizing the separation of the inner sheath 120 from the outer sheath 150.

It should be noted that a predetermined angle that the sliding member 140 rotates in the third direction 500 mentioned above refers to the angle that the sliding member 140 rotates when moving from the second position to the end of the third sliding portion 233.

In the present disclosure, two sliding members 140 are provided, two guide slots 234 are provided, and the two guide slots 234 are symmetrically disposed on the guide member 230. As a result, the predetermined angle that the sliding members 140 rotate in the third direction 500 is less than 180°.

In other embodiments, three, four or even more sliding members 140 are provided, and the number of guide slots 234 is the same as the number of sliding members 140. In this case, a central angle corresponding to the total arc formed by the projection of the guide slots 234 along their own axial direction on the second projection plane is inversely proportional to the specific number of guide slots 234, and the central angle is the predetermined angle that the sliding members 140 rotate in the third direction 500.

The above is the docking process between the drive assembly 200 and the ultrasound probe 100. It can be understood that the separation and locking of the drive assembly 200 and the ultrasound probe 100 are reverse processes, and the specific steps of separation will not be repeated here.

The technical features of the above embodiments can be combined arbitrarily. To make the description concise, not all possible combinations of the technical features in the above embodiments are described. However, as long as there is no contradiction in the combination of these technical features, they should be regarded as the scope of the present specification.

The above embodiments only express several implementation modes of the present disclosure, and their descriptions are specific and detailed, but they cannot be understood as limitations on the scope of the patent application. It should be noted that for those of ordinary skill in the art, without departing from the concept of the present disclosure, several modifications and improvements can be made, which all fall within the protection scope of the present disclosure. Therefore, the protection scope of the patent of the present disclosure should be subject to the appended claims.

## Claims

1. An interventional ultrasound imaging device, comprising:
an ultrasound probe (100) comprising a first connector (110), an inner sheath (120) and a housing (130) sequentially sleeved from inside to outside, wherein the housing (130) is provided with a sliding member (140); and
a drive assembly (200) comprising a second connector (210), a fixing member (220) and a guide member (230) sequentially sleeved from inside to outside, wherein the guide member (230) is provided with a guide slot (234);
wherein the device has a separated state where the first connector (110) is separated from the second connector (210), and a locked state where the first connector (110) is electrically connected to the second connector (210) and the inner sheath (120) extends into the fixing member (220) partially and is connected to the fixing member (220); and
wherein the sliding member (140) is configured to slide along the guide slot (234) to switch the device between the separated state and the locked state.

2. The device according to claim 1, wherein a through hole (222) is disposed on the fixing member (220), a movable member (240) is movably disposed in the through hole (222), and a limiting groove (121) is disposed on the inner sheath (120); and
wherein when the device is in the locked state, the movable member (240) is partially located in the through hole (222) and partially located in the limiting groove (121) to connect the fixing member (220) and the inner sheath (120).

3. The device according to claim 2, wherein the drive assembly (200) comprises a crimping member (250), the crimping member (250) is disposed between the guide member (230) and the fixing member (220), and the crimping member (250) is configured to restrict the movable member (240) from being disengaged from the through hole (222).

4. The device according to claim 3, wherein the through hole (222) comprises a large end and a small end, the large end faces the crimping member (250), and the small end faces the second connector (210); and
a pressing portion (251) is disposed on the crimping member (250), and the pressing portion (251) is attached onto a side of the movable member (240) away from the second connector (210), so that the movable member (240) is partially disposed in the through hole (222) and partially disposed in the limiting groove (121).

5. The device according to claim 4, wherein the ultrasound probe (100) is capable of approaching the drive assembly (200) in a first direction to dock the ultrasound probe (100) with the drive assembly (200), and the guide slot (234) comprises a first sliding portion (231) extending parallel to the first direction;
an abutment groove (252) is disposed at an end of the crimping member (250) close to the ultrasound probe (100), and a length of the first sliding portion (231) in the first direction is greater than a length of the abutment groove in the first direction; and
the sliding member (140) is capable of extending through the first sliding portion (231) and the abutment groove (252) simultaneously and abuts against a groove wall of the abutment groove (252), and the sliding member (140) is capable of sliding along the first sliding portion (231) to push the crimping member (250) to move in the first direction, so that the pressing portion (251) is separated from the movable member (240).

6. The device according to claim 5, wherein the drive assembly (200) further comprises a reset member (260), a protrusion (221) is disposed on the fixing member (220), the reset member (260) is sleeved on the fixing member (220), an end of the reset member (260) abuts against the pressing portion (251), and another end of the reset member (260) abuts against the protrusion (221); and
wherein the reset member (260) is capable of pushing the crimping member (250) to move in a second direction through an elastic force of the reset member (260), and the second direction is opposite to the first direction.

7. The device according to claim 6, wherein the guide slot (234) comprises a second sliding portion (232) communicating with the first sliding portion (231), and the second sliding portion (232) extends in a circumferential direction of the guide member (230);
wherein an inclined surface groove (253) communicating with the abutment groove (252) is disposed on an outer peripheral surface of the crimping member (250), and a groove wall of the inclined surface groove (253) extends from the groove wall of the abutment groove (252) in a direction away from the ultrasound probe (100); and
wherein the sliding member (140) is capable of extending through the second sliding portion (232) and the inclined surface groove (253) simultaneously and sliding along the second sliding portion (232), so that the reset member (260) pushes the pressing portion (251) to move in the second direction.

8. The device according to claim 7, wherein a projection of the inclined surface groove (253) in an axial direction of the crimping member (250) on a first projection plane forms a first arc, an arc length of the first arc is less than an arc length of the second sliding portion (232), and the first projection plane is a projection plane of the crimping member (250) that is perpendicular to the axial direction of the crimping member (250).

9. The device according to claim 7 or 8, wherein the ultrasound probe (100) comprises an outer sheath (150) and a damping member (160), the outer sheath (150) is connected to the housing (130), the damping member (160) is sleeved on the inner sheath (120), and the outer sheath (150) is connected to the inner sheath (120) through the damping member (160).

10. The device according to claim 9, wherein a recessed portion (254) communicating with the inclined surface groove (253) is disposed on an outer peripheral surface of the crimping member (250);
wherein the guide slot (234) comprises a third sliding portion (233) communicating with the second sliding portion (232), and the third sliding portion (233) extends from an end of the second sliding portion (232) away from the first sliding portion (231) in a direction close to the ultrasound probe (100); and
wherein the sliding member (140) extends through the third sliding portion (233) and the recessed portion (254), and slides in a direction away from the second sliding portion (232) to separate the damping member (160) from the outer sheath (150).

11. The device according to claim 10, wherein the sliding member (140) extends through the third sliding portion (233) and the recessed portion (254), and slides in a direction close to the second sliding portion (232) to connect the damping member (160) to the outer sheath (150).

12. The device according to claim 11, wherein a length of the third sliding portion (233) in the first direction is greater than or equal to a predetermined movement distance of the outer sheath (150).

13. The device according to any one of claims 4 to 12, wherein the drive assembly (200) further comprises a retaining ring (270), the retaining ring (270) is sleeved on the fixing member (220) and disposed on a side of the movable member (240) close to the ultrasound probe (100), and the retaining ring (270) abuts against the pressing portion (251) when the pressing portion (251) is attached onto the movable member (240).

14. The device according to any one of claims 1 to 13, wherein the ultrasound probe (100) further comprises a first rotating shaft (170) extending through the inner sheath (120), the first connector (110) is mounted on the first rotating shaft (170), and a first transmission member (180) is mounted on the first rotating shaft (170);
wherein the drive assembly (200) further comprises a second rotating shaft (280) extending through the fixing member (220), the second connector (210) is mounted on the second rotating shaft (280), and a second transmission member (290) is mounted on the second rotating shaft (280); and
when the device is in the locked state, the second transmission member (290) is configured to contact the first transmission member (180), so that the drive assembly (200) drives the ultrasound probe (100) to rotate.

15. The device according to claim 14, wherein the first transmission member (180) comprises a first abutment plane (181), the second transmission member (290) comprises a second abutment plane (291), and the first abutment plane (181) is configured to be attached onto the second abutment plane (291).
